(19) Europäisches Patentamt
European Patent Office
Office européen des brevets

(11) Publication number: 0 005 004
B1

(12) EUROPEAN PATENT SPECIFICATION

(45) Date of publication of patent specification: 16.02.83

(21) Application number: 79300303.9

(22) Date of filing: 28.02.79

(51) Int. Cl.³: C 07 H 15/04, C 07 H 15/08, C 07 H 15/10 //C12P19/12

# (54) Hydroxyalkyl-etherified glycolipid esters and their preparation.

(30) Priority: 03.03.78 JP 24306/78

(43) Date of publication of application: 31.10.79 Bulletin 79/22

(45) Publication of the grant of the patent: 16.02.83 Bulletin 83/7

(84) Designated Contracting States: BE CH DE FR GB IT LU NL SE

(56) References cited:
GB - A - 2 002 369

JOURNAL OF ORGANIC CHEMISTRY, vol. 37, no. 18, September 8, 1972 by American Chemical Society, A. P. TULLOCH et al.: "Formation of a Long-Chain Alcohol Ester of Hydroxy Fatty Acid Sophoroside by Fermentation of Fatty Alcohol by a Torulopis Species", pages 2868—2870

(73) Proprietor: KAO SOAP COMPANY, LIMITED
Nihonbashi-Kayabacho 1-chome Chuo-ku
Tokyo (JP)

(72) Inventor: Inoue, Shigeo
22-6, Miyashirodai Miyashiro-cho
Minami-Saitama-gun Saitama-ken (JP)
Inventor: Kimura, Yoshiharu
Kao-Shataku 1-202 Nr 2606-6 Ohaza Akabane
Ichikai-machi Haga-gun Tochigi-ken (JP)
Inventor: Kinta, Manzo
8, Gyoda-cho Funabashi-shi
Chiba-ken (JP)

(74) Representative: Webb, Frederick Ronald et al,
G.F. Redfern & Co. Marlborough Lodge 14
Farncombe Road
Worthing West Sussex BN11 2BT (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 005 004 B1

Hydroxyalkyl-etherified glycolipid esters and their preparation

This invention relates to novel wax-like substances possessing surface activity characteristics, and to a process for producing the same. More particularly, the invention relates to novel hydroxyalkyl-etherified glycolipid esters.

Known waxes (also in some cases called greases) include esters of a long-chain fatty acid and a long-chain aliphatic alcohol, long-chain hydrocarbons, glycerides, lanolin and similar materials. The esters have been widely used for various purposes, such as glazing materials and oily substances for leather, in cosmetics and ointments, as lubricants for fibres, in metal processing, machinery, stationery, and printing oils, for rust inhibition and for insulation. In recent years, wax has often been used in emulsified form with a surface active agent in view of its safety and ease of handling. However, formulation techniques of a high quality are required for the emulsification of wax because the wax characteristics depend directly upon the formulation technique used. Only extremely high quality formulation techniques permit wax to exhibit all of its inherent characteristics, but such techniques involve much difficulty. Hence, there has been a strong desire for the development of a wax-like substance which itself possesses surface activity.

Wool wax, which is generally known under the name of lanolin, includes in its structure a particular ester bond formed between the long-chain branched fatty acid and the long-chain branched aliphatic alcohol or steroid alcohol, and hence it is a waxy substance having both excellent wax-like properties and an adequate emulsifying ability. However, wool wax is a mysterious, naturally occurring compound and finds no wide application, partly because its combination with other materials requires the use of very advanced techniques and partly because the amounts used are limited due to its peculiar animal smell.

In view of this situation, the present Applicants have carried out a wide variety of studies on the development of a compound possessing both surface activity and wax-like properties, and have found that a glycolipid ester represented by the following formula (III) exhibits the desired activity and properties:—

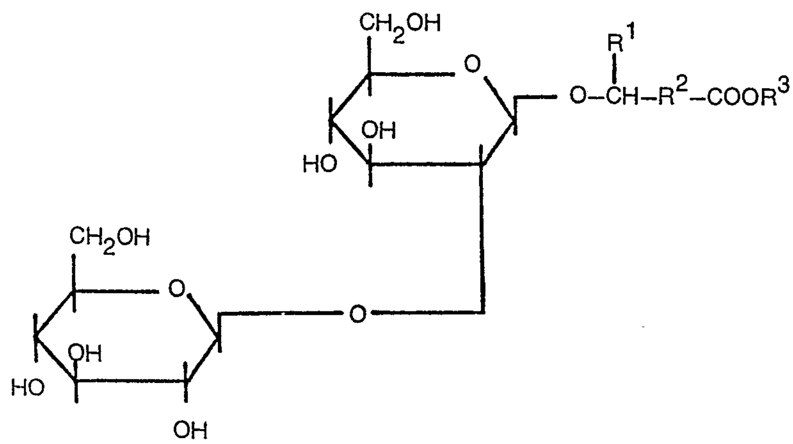

(III)

where $R^1$ is a methyl group or a hydrogen atom, $R^2$ is a straight-chain saturated or ethylenically unsaturated hydrocarbon group having 11 to 15 carbon atoms when $R^1$ is a methyl group, or $R^2$ is a straight-chain saturated or ethylenically unsaturated hydrocarbon group having 12 to 16 carbon atoms when $R^1$ is a hydrogen atom, and $R^3$ is a saturated or unsaturated hydrocarbon group having 1 to 20 carbon atoms.

However, the glycolipid ester of the formula (III) has been found to lack sufficient heat stability at high temperatures and viscosity. The reason is that the hydroxyl groups of the sugar moiety of the glycolipid ester exist in a free form, thereby resulting in reduced adhesiveness, lubricity, flexibility and plasticity.

A further study has therefore been made in order to improve the above-mentioned properties arising from the sugar structure, and as a result, various derivatives of the glycolipid ester of the formula (III) have been synthesised by chemical modification of the hydroxyl groups. After investigating the characteristics of these derivatives, it has been found that certain compounds obtained by modification of the hydroxyl groups with hydroxyalkyl groups, possess the above desired characteristics, viz. wax-like properties and surface activity, but with increased heat stability.

It is, therefore, an object of this invention to provide novel hydroxyalkyl-etherified glycolipid esters possessing excellent surface activity and wax-like properties, but having increased heat stability.

It is another object of the invention to provide a novel process for producing such esters.

According to the invention, there are provided novel hydroxyalkyl-etherified glycolipid esters of the Formula (I):—

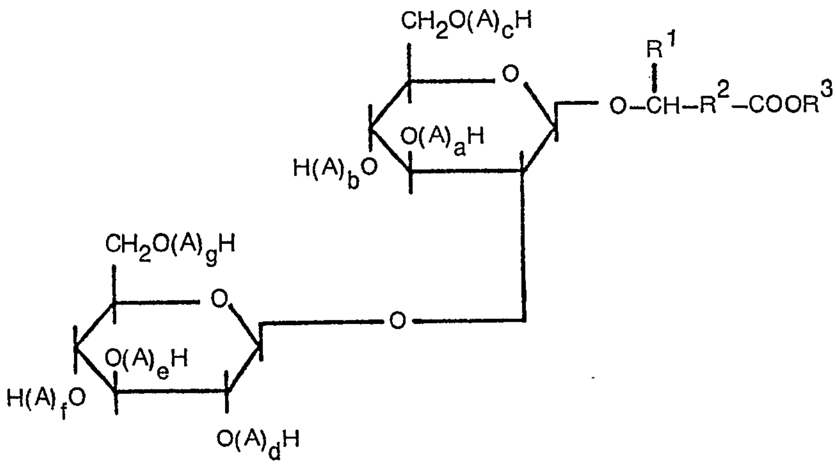

(I)

where $R^1$ is a methyl group or a hydrogen atom, $R^2$ is a straight-chain saturated or ethylenically unsaturated hydrocarbon group having 11 to 15 carbon atoms when $R^1$ is a methyl group, or a straight-chain saturated or ethylenically unsaturated hydrocarbon group having 12 to 16 carbon atoms when $R^1$ is a hydrogen atom, $R^3$ is a saturated or unsaturated hydrocarbon group having 1 to 20 carbon atoms, A is $—CH_2—CH_2—O—$ or

$$\overset{CH_3}{\underset{|}{}}\\ —CH_2—CH—O—,$$

and a, b, c, d, e, f and g are integers, whose sum ranges from 1 to 60 (particularly 5 to 30), and of formula (II):—

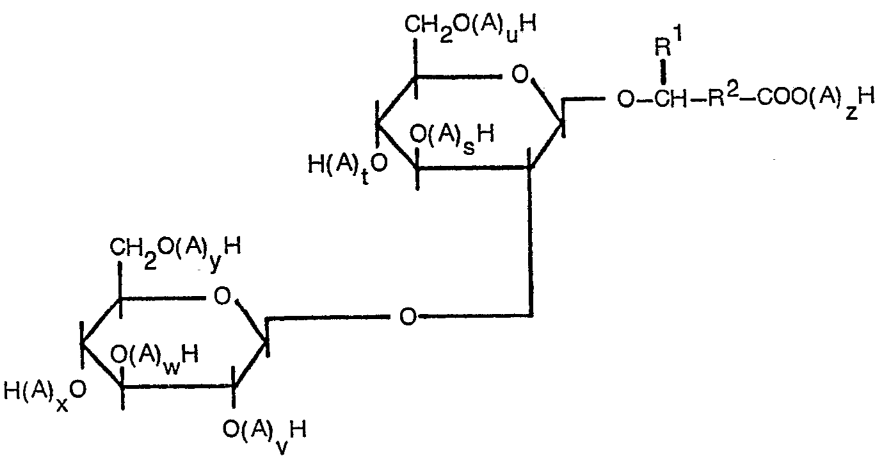

(II)

where $R^1$, $R^2$ and A are the same as defined above, and s, t, u, v, w, x, y and z are integers, whose sum ranges from greater than 1 to 60 (particularly 5 to 30), not all of the integers s to y being zero, z not being zero, and not all of the integers s to z having the value 1.

The hydroxyalkyl-etherified glycolipid esters of the formulae (I) and (II) are produced according to the present invention, as shown in the following reaction schemes by reacting a glycolipid ester of the formula (III) or a glycolipid of the formula (IV) respectively with ethylene oxide or 1,2-propylene oxide in the presence of an alkylene oxide addition catalyst.

(Reaction Equation I)

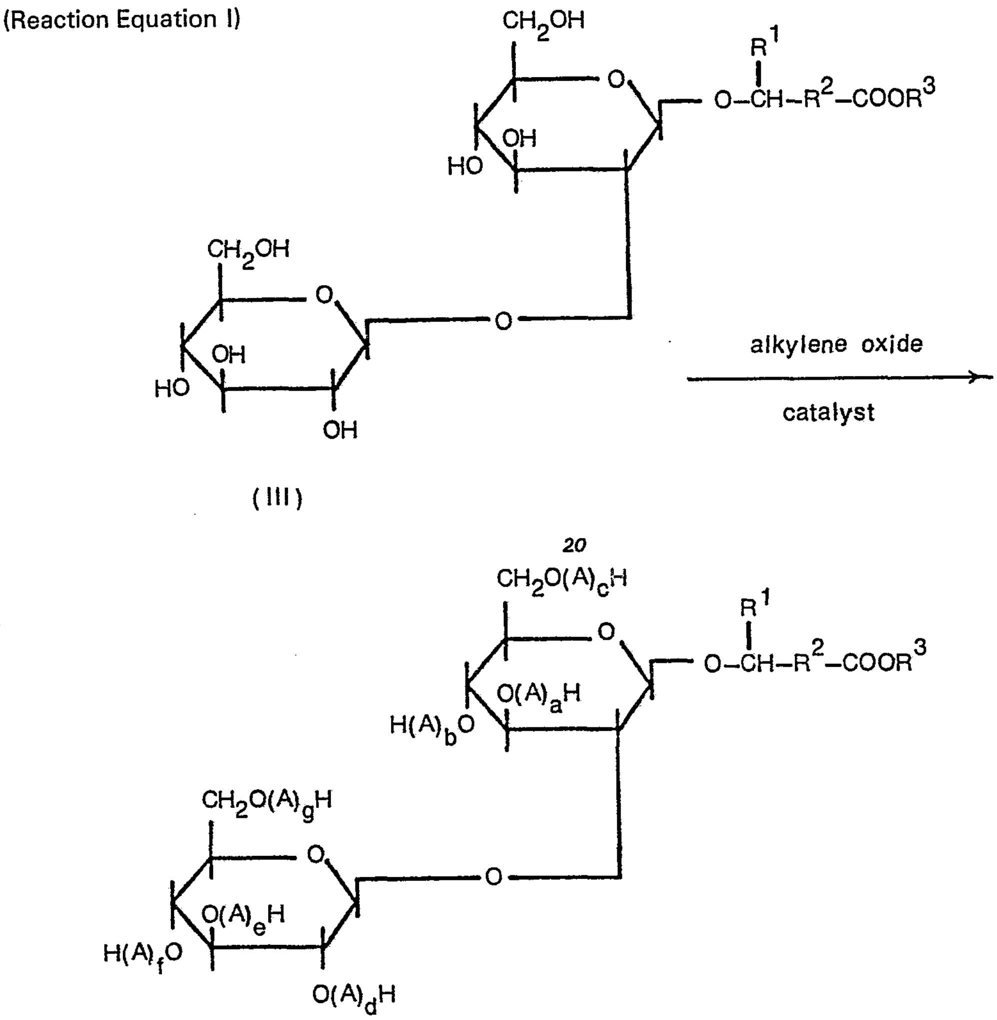


(III)

(I)

In the formula (III), $R^1$, $R^2$, $R^3$ and A are as in formula (I).

(Reaction Equation II)

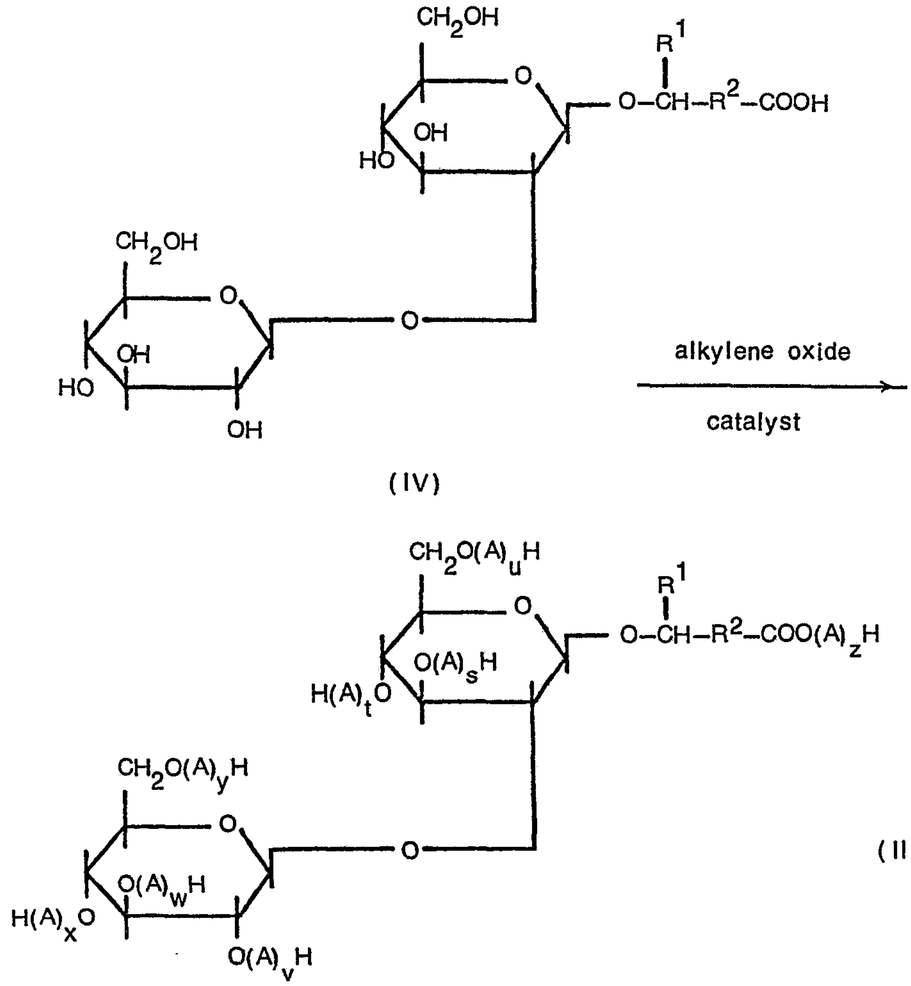


(IV)

(II)

In the formula (IV), $R^1$, $R^2$ and A are the same as in formula (II), and s, t, u, v, w, x, y and z represent integers, whose sum ranges from 1 to 60, not all the integers s to y being zero, z not being zero, and not all the integers s to z having the value 1.

The glycolipid ester starting material having the formula (III) can be produced by a method discovered by the present Applicants. Sophorolipid is first subjected to methanolysis and methylation reactions by reaction with methanol in an acid to produce a glycolipid methyl ester (see Japanese Patent Application No. 1978—17401), which is further converted into other compounds of the formula (III) by an ester interchange reaction with an alcohol of the formula, ROH, where R is a saturated or unsaturated hydrocarbon group having 2 to 20 carbon atoms, (see Japanese Patent Application No. 1978—17400). The glycolipid starting material of the formula (IV) is produced by hydrolysing the glycolipid methyl ester.

It has been reported by J. F. T. Spencer et al [Canadian Journal of Chemistry, *39*, 846 (1961)] that a great quantity of Sophorolipid is produced by culturing *Torulopsis bombicola*.

Sophorolipid is a mixture of the compounds represented by the formulae (Va) and (Vb),

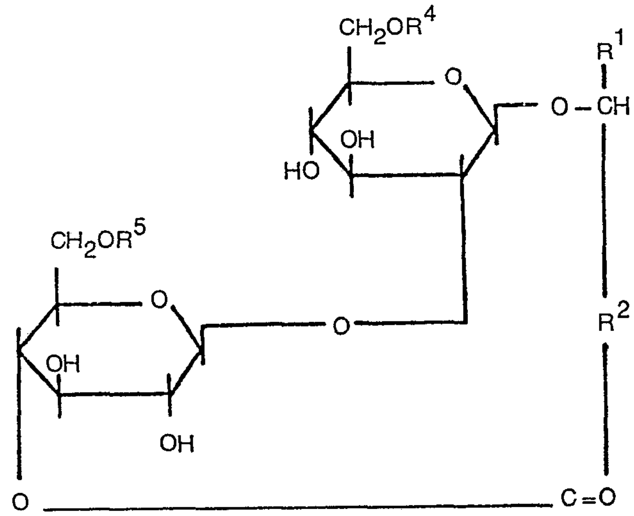

(Va)

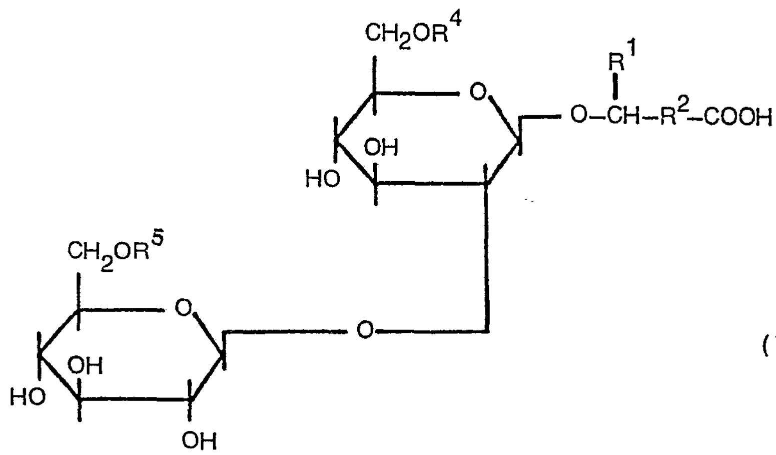

(Vb)

wherein $R^1$ and $R^2$ are as defined above and $R^4$ and $R^5$ can represent the following combinations of groups:—

Va-1 : $R^4 = R^5 = COCH_3$
Va-2 : $R^4 = COCH_3$, $R^5 = H$
Va-3 : $R^4 = H$, $R^5 = COCH_3$
Va-4 : $R^4 = R^5 = H$
Vb-1 : $R^4 = R^5 = COCH_3$
Vb-2 : $R^4 = COCH_3$, $R^5 = H$
Vb-3 : $R^4 = H$, $R^5 = COCH_3$
Vb-4 : $R^4 = R^5 = H$.

As can be seen from the above formulae (Va) and (Vb), Sophorolipid is a mixture of many glycolipids; its basic structure is that of a [(2′-O-β-D-glucopyranosyl-β-D-glucopyranosyl)-oxy]-alkane acid or alkene acid which is formed by a glucoside bond between Sophorose and a long-chain fatty acid having a hydroxy group at the ω or ω-1 position.

The glycolipid esters of the invention are produced by reacting ethylene oxide or 1,2-propylene oxide with the starting materials produced by the above-mentioned processes.

In general, the compounds of the formulae (III) and (IV) readily react with alkylene oxides under any conditions applicable to normal alkylene oxide addition reaction. However, an alkaline alkylene oxide addition catalyst is preferably used because glucoside bonds of the disaccharide may be cleaved by an acid.

Suitable alkylene oxide addition catalysts which can be used in the invention include metallic sodium metallic potassium, sodium hydroxide, potassium hydroxide, sodium alcoholates, potassium alcoholates, and the like. Suitable alcohols which can be used to prepare the sodium or potassium

alcoholates are methanol, ethanol and isopropanol. Any one selected catalyst is preferably used in an amount of from 0.1 to 1.0% by weight of the glycolipid or glycolipid ester. The reaction is preferably carried out at a temperature in the range of from 70 to 120°C, especially from 100 to 120°C. The catalysts may be used singly or in combination.

The reaction progress can be observed by measuring the pressure in the reactor or by quantitatively analysing the reaction products so that an adduct having any predetermined addition number can be produced.

The hydroxyalkyl-etherified glycolipid ester according to the invention thus obtained, did not undergo any structural degeneration or deterioration in a preservation test at 250°C for 48 hours, because the hydroxyl groups in the sugar residues are combined with the hydroxyalkyl groups through stable ether bonds, and thus had increased heat stability as compared with the unetherified ester. Moreover, a variety of substances having both wax-like and hydrophilic properties and surface activities suitable for any desired purposes of application can be produced by varying the type of glycolipid ester used and/or the addition mole number since any addition mole number of the alkylene oxide within the range of 1 to 60 may be specified.

The characteristics of some typical compounds of the formulae (I) and (II) are shown in Table 1 below, in which EO and PO denote ethylene oxide and propylene oxide respectively:—

TABLE 1

| Ester Residue | Adduct | Characteristics |
|---|---|---|
| $CH_3$ | EO | Readily soluble in water. HLB value is of the order of 20. Greatly effective as a cleanser. With 30 moles of adduct is fairly soluble in hydrocarbon solvents and mixes well with waxy substances. Suitable as an auxiliary agent for lubrication and softening by wax. |
| $C_{12}H_{25}$ | EO | Soluble in warm water. Emulsified and dispersed at low temperature. Wax-like substance having an HLB value of 13 to 15. Has a high mixing ability with waxy substances. Useful as a softening lubricant for fibres and as an emulsifying agent for an emulsified type of glazing material and a waxy agent. Has an especially desirable effect when used as a moisturiser for cosmetics. |
| $C_{18}H_{37}$ | EO | Viscous pasty substance having properties similar to lanolin. It is an oil with a fibrous structure and has a good mixing ability with waxy substances. Can be used as an emulsifying agent, a glazing material, a leather oil, a softening lubricant for fibres, an oil for use in cosmetics and ointments, especially as a moisturiser for ointments and cosmetics, when it has a particularly desirable effect. |
| Oxyethylene | EO | Readily soluble in water. HLB value is more than 20. Effective as a cleanser. Exerts an enhancing effect on a lubricant and a softening agent. Effective as a moisturiser for cosmetics. |
| $CH_3$ | PO | Slightly soluble in water. Viscous paste. Can be emulsified and dispersed on a warm bath. Has an excellent mixing ability with higher fatty acids, higher alcohols and the like, and a good adhesion to the surface of solids. Can be used as a fat-addition agent and a softening lubricant for leather, skin and fibres. |
| $C_{12}H_{25}$ | PO | Only slightly soluble in water. Grease-like paste having an extensibility and lubricating ability. Mixes well with hydrocarbon solvents and homogeneously with various kinds of wax. When used with wax, the properties of the wax are enhanced because it lowers the viscosity of solid wax and glycerides, and softens them. |

TABLE 1 (Continued)

| Ester Residue | Adduct | Characteristics |
|---|---|---|
| $C_{18}H_{37}$ | PO | Insoluble in water. Elastic paste-like grease. Has an extremely good mixing ability with hydrocarbon solvents and wax. Has wide application as a quality-improving agent for wax, a fat-addition agent, a softening lubricant, an oil for cosmetics and ointments, an oil for metal processing, a machine oil, a rust inhibitor, an oil for stationery and an oil for printing. |
| Oxypropylene | PO | Only slightly soluble in water. Viscous paste. Emulsified and dispersed on a warm bath. Has an excellent mixing ability with higher fatty acids, higher alcohols and the like, and a good adhesion to the surface of solids. Effective as a fat-addition agent and a softening lubricant for leather, skin and fibre. |

When ethylene oxide is used, A in the above formulae is $—CH_2.CH_2.O—$, and when propylene oxide is used, A is $—CH_2—CH(CH_3).O—$.

It is apparent from the above Table that the compounds of the formula (I) possess an extremely excellent effect when applied as an oil for cosmetics and leather, and as a softening lubricant for fibres. Especially when used in an amount of from 2 to 10% by weight for cosmetics, the compounds serve as emulsifying agents, and impart elasticity and lubricity to the skin because of their affinity therefor.

The present invention is illustrated by the following non-limitative Examples:—

General Example of the Preparation of Starting Materials

(i) To a mixture of 1500 g of glucose, 75 g of a yeast extract and 15 g of urea water was added to adjust the whole volume to 15 l, and the resulting mixture was sterilised and used as a fermentation liquid. This liquid was inoculated with *Torulopsis bombicola* which had been cultured on the same medium as above at 30°C for 48 hours. The fermentation was started with stirring at a speed of 300 rpm and aerated at 0.33 VVM at 20°C. The culture was carried on for 24 hours after inoculation of the micro-organism, and 150 g of a tallow oil was added at intervals of 24 hours. The added tallow oil amounted to 900 g. After the final addition, the culture was continued for 24 hours. The total culture time amounted to 168 hours. A Sophorolipid layer which precipitated on the bottom of the fermentation vessel was collected by decantation to give 1300 g of Sophorolipid in paste form at room temperature which had a water content of about 50%.

(ii) 100 g of the Sophorolipid thus obtained and 2.5 g of polypropylene glycol having an average molecular weight of 200 were placed in a 200 ml round bottom flask equipped with a stirrer and a Liebig condenser. The mixture was evaporated with stirring at 80°C in an oil bath under a reduced pressure of 0.333 bar (250 mmHg) to eliminate water. After evaporation for about 2 hours, water was completely removed by distillation, and the water content was found to be less than 1%.

(iii) To the Sophorolipid-polypropylene glycol solution thus obtained, were added 150 g of methanol and then 2.5 g of sulphuric acid. The resulting mixture was maintained at 40°C ± 2°C for 90 minutes. The progress of the methanolysis-methylation reaction was observed by thin layer-chromatography on silica gel [solvent : chloroform-methanol-acetic acid (75 : 20 : 5)], and the reaction was regarded as having reached completion when many spots shown by the starting material or Sophorolipid converged on the thin layer chromatograph.

After the completion of the reaction, the reaction solution was neutralised with potassium hydroxide and filtered through filter paper. The filtrate was placed in a round-bottom flask equipped with a Liebig condenser, and methanol and methyl acetate were distilled off to give 48 g of a brown pasty mixture which contained 94% of [(2′-O-β-D-glucopyranosyl-β-D-glucopyranosyl)-oxy]-alkane acid and -alkene acid methyl esters, and polypropylene glycol. This mixture was purified by column-chromatography on silica gel, and there were obtained pure [(2′-O-β-D-glucopyranosyl-β-D-glucopyranosyl)oxy]-alkane acid and -alkene acid methyl esters.

IR ($cm^{-1}$) :

1740 ( >C=O ester)

1380~3200 (—OH sugar)
900~750 (glucopyranose ring)

NMR [δ(pyridine)] : 1.1~1.6 ($—CH_2—CH_2—$)
3.6 ($—O—CH_3$)
3.5~5.0 (sugar)
5.5 (—CH=CH— unsaturated fatty acid)

Oil Characterisation Analyses:

| | |
|---|---|
| Acid value | : 0 |
| Hydroxy value | : 615 |
| Saponification value | : 88 |
| Ester value | : 87. |

This product was degraded in a 5 N hydrochloric acid-methanol solution to give 2 moles of a methyl glucoside and 1 mole of a hydroxy fatty acid methyl ester, which were quantitatively analysed by gas chromatography.

### Example 1

100 g of a mixture of [(2′-O-β-D-glucopyranosyl-β-D-glucopyranosyl)oxy]-alkane acid and -alkene acid methyl esters and polypropyleneglycol obtained as in the General Example, was placed in an autoclave together with 0.25 g of potassium hydroxide, and the mixture was reacted at 100—120°C for 6 hours with various amounts of ethylene oxide gas corresponding to the addition mole numbers shown in Table 2 which were passed into the reaction mass. After the completion of the reaction, the mixture was neutralised with phosphoric acid, and potassium phosphate which formed was removed by filtration under increased pressure to obtain a crude product. This product was purified by column-chromatography on silica gel to remove an ethylene oxide adduct of polypropylene glycol, thereby yielding polyoxyethylene[(2′-O-β-D-glucopyranosyl-β-D-glucopyranosyl)oxy]-alkane acid and -alkene acid methyl esters as a pale yellow paste.

TABLE 2

| Addition mole number | Amount of ethylene oxide used (g) | Crude product (g) |
|---|---|---|
| 5 | 34.4 | 132 |
| 7 | 48.2 | 145 |
| 15 | 103.3 | 200 |
| 30 | 206.6 | 305 |

IR ($cm^{-1}$) : 3380~3200 (—OH)
1740 (>C=O ester)
1100 (—O—)

TABLE 3

Oil Characterisation Analyses

| Moles of Adduct | Hydroxyl value | Acid value | Saponification value | Ester value |
|---|---|---|---|---|
| 5 | 463.5<br>(458.4) | 0.5<br>(0) | 67.0<br>(65.5) | 66.5<br>(65.5) |
| 7 | 420.1<br>(415.7) | 1.2<br>(0) | 60.9<br>(59.4) | 59.7<br>(59.4) |
| 15 | 311.3<br>(302.9) | 0.7<br>(0) | 45.2<br>(43.3) | 44.5<br>(43.3) |
| 30 | 205.8<br>(200.7) | 0.7<br>(0) | 30.0<br>(28.7) | 29.3<br>(28.7) |

(In this and subsequent Oil Characterisation Analyses Tables, the value without brackets is the measured value and that in brackets, the calculated value).

Example 2

100 g of a mixture of [(2′-O-β-D-glucopyranosyl-β-D-glucopyranosyl)oxy]-alkane acid and -alkene acid methyl esters and polypropylene glycol (obtained as in the General Example) was placed in an autoclave, together with 0.25 g of potassium hydroxide, and the mixture was reacted at 100—120°C for 6 hours with amounts of propylene oxide gas corresponding to the addition mole numbers shown in Table 4, which were passed into the reaction mass. After the completion of the reaction, in each case, the mixture was neutralised with phosphoric acid and filtered under increased pressure to obtain a crude product as a brown paste. This product was purified by column-chromatography on silica gel to give polyoxypropylene [(2′-O-β-D-glucopyranosyl-β-D-glucopyranosyl)oxy]-alkane acid and -alkene acid methyl esters as a pale yellow paste.

TABLE 4

| Addition mole number | Amount of propylene oxide used (g) | Crude product (g) |
|---|---|---|
| 5 | 45.4 | 143 |
| 7 | 63.55 | 158.5 |
| 15 | 136.2 | 230 |
| 30 | 272.4 | 365 |

IR ($cm^{-1}$) : 3380~3200 (—OH)

1740 (>C=O ester)

1100 (—O—)

TABLE 5

Oil Characterisation Analyses

| Moles of Adduct | Hydroxyl value | Acid value | Saponification value | Ester value |
|---|---|---|---|---|
| 5 | 430.1<br>(423.8) | 0.8<br>(0) | 62.6<br>(60.5) | 61.8<br>(60.5) |
| 7 | 382.3<br>(376.7) | 0.7<br>(0) | 53.7<br>(53.8) | 53.0<br>(53.8) |
| 15 | 271.0<br>(260.7) | 1.0<br>(0) | 39.5<br>(37.2) | 38.5<br>(37.2) |
| 30 | 170.7<br>(165.3) | 0.7<br>(0) | 25.3<br>(23.6) | 24.6<br>(23.6) |

Example 3

(i) 100 g of a mixture of [(2′-O-β-D-glucopyranosyl-β-D-glucopyranosyl)oxy]-alkane acid and -alkene acid methyl esters, and polypropylene glycol (obtained as in the General Example), 30 g of water and 70 g of ethanol were placed in a 500 ml round-bottom flask. The mixture was stirred to obtain a homogenous solution. To this solution was added 9 g of potassium hydroxide, and the resulting mixture was reacted under reflux conditions for 2 hours. After the completion of the reaction, the mixture was neutralised with sulphuric acid, and potassium sulphate which formed was filtered off. The filtrate was placed in a 500 ml three-neck flask equipped with a Liebig condenser and was evaporated under normal pressure to remove methanol and ethanol. Water was then distilled off under reduced pressure to yield [(2′-O-β-D-glucopyranosyl-β-D-glucopyranosyl)oxy]-alkane acid and -alkene acid.

(ii) The product obtained by process (i) above was placed in an autoclave together with 0.25 g of potassium hydroxide, and the mixture was reacted at 100—120°C for 6 hours with amounts of propylene oxide gas corresponding to the addition mole numbers shown in Table 6 below, which were passed into the reaction mass. After the completion of the reaction, the mixture was neutralised with phosphoric acid and filtered under increased pressure to obtain a brownish viscous crude product. This product was purified by column-chromatography on silica gel to give polyoxypropylene [(2′-O-β-D-glucopyranosyl-β-D-glucopyranosyl)oxy]-alkane acid and -alkene acid esters as a pale yellow paste.

TABLE 6

| Addition mole number | Amount of propylene oxide (g) | Crude Product (g) |
|---|---|---|
| 5 | 45.2 | 143 |
| 8 | 72.2 | 168 |
| 15 | 135.4 | 230 |
| 30 | 270.8 | 366 |

IR ($cm^{-1}$) : 3380~3200 (—OH)

1740 (>C=O ester)

1100 (—O—).

TABLE 7

Oil Characterisation Analyses.

| Moles of Adduct | Hydroxyl value | Acid value | Saponification value | Ester value |
|---|---|---|---|---|
| 5 | 501.2<br>(491.9) | 0.9<br>(0) | 63.2<br>(61.5) | 62.3<br>(61.5) |
| 8 | 421.4<br>(412.8) | 0.7<br>(0) | 53.4<br>(51.6) | 52.7<br>(51.6) |
| 15 | 305.3<br>(299.1) | 0.7<br>(0) | 38.9<br>(37.4) | 38.2<br>(37.4) |
| 30 | 182.0<br>(188.0) | 0.3<br>(0) | 24.8<br>(23.5) | 24.5<br>(23.5) |

Example 4

Addition of ethylene oxide to glycolipid octyl ester:

20 g of a glycolipid methyl ester-polypropylene glycol mixture was placed in an autoclave, and to this mixture were added 4.2 g of octyl alcohol and 5 g of methanol to obtain a homogenous solution, and then 0.1 g of sodium methylate. The greater part of the methanol was removed by distillation at 70°C, and an ester interchange reaction was carried out while methanol which formed was distilled off with stirring under a reduced pressure of 0.333 bar (250 mmHg). The progress of the reaction was observed by gas-chromatography under the following conditions: glass column; 3% silicon JXR—Chromosorb W having a 60~80 mesh per centimetre particle size; inside diameter 3 mm; height 1 m; column oven temperature of 250~350°C; pressure by helium gas of 0.6 kg/cm$^2$; and hydrogen flame detector used as a detector. The reaction state was measured from the peak area ratio of the glycolipid methyl ester and glycolipid octyl ester with a sample picked up from the reaction solution and trimethylsilylated with a trimethylsilylating agent. The reaction was regarded as having been completed when the peak of the glycolipid methyl ester disappeared. The mixture was reacted at 100—120°C for 6 hours with ethylene oxide gas in the amounts specified in Table 8, the gas being passed into the reaction mass, to obtain an ethylene oxide adduct. The amount of ethylene oxide was varied according to the specified addition mole number as shown in Table 8.

TABLE 8

| Addition mole number | Amount of ethylene oxide (g) | Crude product (g) |
|---|---|---|
| 7 | 9.8 | 33.5 |
| 15 | 20.9 | 44.6 |
| 30 | 41.8 | 65.0 |

The mixture was neutralised with phosphoric acid, and sodium phosphate which formed was filtered off under increased pressure. 5 g of the sample was purified by column-chromatography on 500 g of silica gel (chloroform-methanol; gradient elution) to obtain polyoxyethylene [(2′-O-β-D-glucopyranosyl-β-D-glucopyranosyl)oxy]-alkane acid and -alkene acid octyl ester fractions which amounted to 91 to 94% by weight of the whole sample weight.

IR (cm$^{-1}$) : 3380~3200 (—OH)

1740 ( 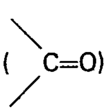

1100 (—O— ether)
1050 (—OH)

TABLE 9

Oil Characterisation Analyses

| Moles of Adduct | Hydroxy value | Acid value | Saponification value | Ester value |
|---|---|---|---|---|
| 7 | (376.5)<br>389.1 | (0)<br>0.7 | (53.8)<br>51.5 | (53.8)<br>50.8 |
| 15 | (281.5)<br>293.8 | (0)<br>0.4 | (40.2)<br>39.0 | (40.2)<br>38.6 |
| 30 | (191.1)<br>200.6 | (0)<br>0.2 | (27.3)<br>26.4 | (27.3)<br>26.2 |

Example 5

Addition of ethylene oxid to glycolipid oleyl ester:

20 g of a mixture of glycolipid methyl esterpolypropylene glycol obtained as in the General Example was placed in an autoclave, and to this mixture were added 8.9 g of oleyl alcohol, 5 g of methanol and then 0.1 g of sodium methylate. The reaction conditions for ester interchange and ethylene oxide addition were the same as in Example 4. 21.0 g of an ethylene oxide gas was reacted with the mixture, thereby obtaining an adduct having an average addition number of 15. 29.8 g of the ethylene oxide adduct thus obtained was neutralised with phosphoric acid, and sodium phosphate which formed was filtered off under increased pressure. 5 g of the sample was purified by column-chromatography under the same conditions as in Example 4 to give 4.5 g of polyoxyethylene [(2'-O-β-D-glucopyranosyl-β-D-glucopyranosyl)oxy]-alkane acid and -alkene acid oleyl ester fractions.

IR ($cm^{-1}$) : 3380~3200 (—OH)

1740 (>C=O)

1100 (—O— ether)

1050 (—OH)

TABLE 10

Oil Characterisation Analyses

| Moles of Adduct | Hydroxyl value | Acid value | Saponification value | Ester value |
|---|---|---|---|---|
| 15 | (255.9)<br>261.1 | (0)<br>0.8 | (36.6)<br>35.0 | (36.6)<br>34.2 |

Example 6

Addition of propylene oxide to glycolipid lauryl ester:

20 g of a mixture of glycolipid methyl esterpolypropylene glycol obtained as in the General Example was placed in an autoclave, and to this mixture were added 6.1 g of lauryl alcohol, 5 g of methanol and then 0.1 g of sodium methylate. The method of preparing a glycolipid lauryl ester was the same as in Example 4. After the completion of the ester interchange reaction, the mixture was reacted at 100 to 120°C for 6 hours with a specified amount of a propylene oxide gas which was passed into the reaction mass, to obtain a propylene oxide adduct. The amount of the propylene oxide gas was varied according to the specified addition mole number as shown in Table 11.

TABLE 11

| Addition mole number | Amount of propylene oxide (g) | Crude product (g) |
|---|---|---|
| 7 | 12.87 | 38.3 |
| 15 | 27.56 | 52.1 |
| 30 | 55.12 | 80.2 |

The propylene oxide adduct thus obtained was neutralised with phosphoric acid, and sodium phosphate which formed was filtered off under increased pressure. 5 g of the sample was purified by column-chromatography under the same conditions as in Example 4 to give polyoxypropylene [(2′-O-β-D-glucopyranosyl-β-D-glucopyranosyl)oxy]-alkane acid and -alkene acid lauryl ester fractions which amounted to 90 to 93% by weight of the whole sample weight.

IR ($cm^{-1}$) : 3380~3200 (—O)

1740 (>C=O)

1100 (—O— ether)

TABLE 12

Oil Characterisation Analyses

| Moles of Adduct | Hydroxyl value | Acid value | Saponification value | Ester value |
|---|---|---|---|---|
| 7 | (328.1)<br>339.4 | (0)<br>0.9 | (46.9)<br>45.7 | (46.9)<br>44.8 |
| 15 | (236.5)<br>247.1 | (0)<br>0.5 | (33.8)<br>32.9 | (33.8)<br>32.4 |
| 30 | (155.2)<br>160.8 | (0)<br>0.3 | (22.2)<br>21.6 | (22.2)<br>21.3 |

**Claims**

1. A hydroxyalkyl-etherified glycolipid ester represented by the formula (I):—

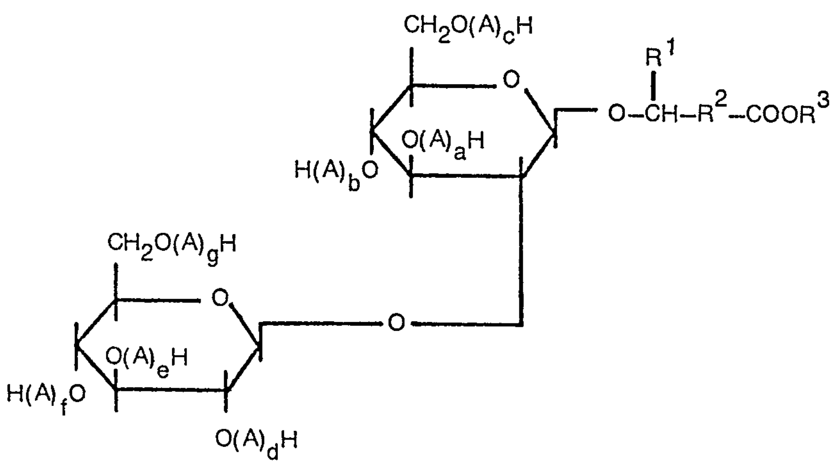

(I)

where $R^1$ is a methyl group or a hydrogen atom, $R^2$ is a straight-chain saturated or ethylenically unsaturated hydrocarbon group having 11 to 15 carbon atoms when $R^1$ is a methyl group, or a straight-chain saturated or ethylenically unsaturated hydrocarbon group having 12 to 16 carbon atoms when $R^1$ is a hydrogen atom, $R^3$ is a saturated or unsaturated hydrocarbon group having 1 to 20 carbon atoms, A is $—CH_2—CH_2—O$ or

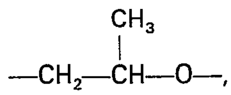

and a, b, c, d, e, f and g are integers, whose sum ranges from 1 to 60.

2. A hydroxyalkyl-etherified glycolipid ester according to Claim 1, characterised in that a, b, c, d, e, f and g are integers, whose sum ranges from 5 to 30.

3. A hydroxyalkyl-etherified glycolipid ester represented by the formula (II):

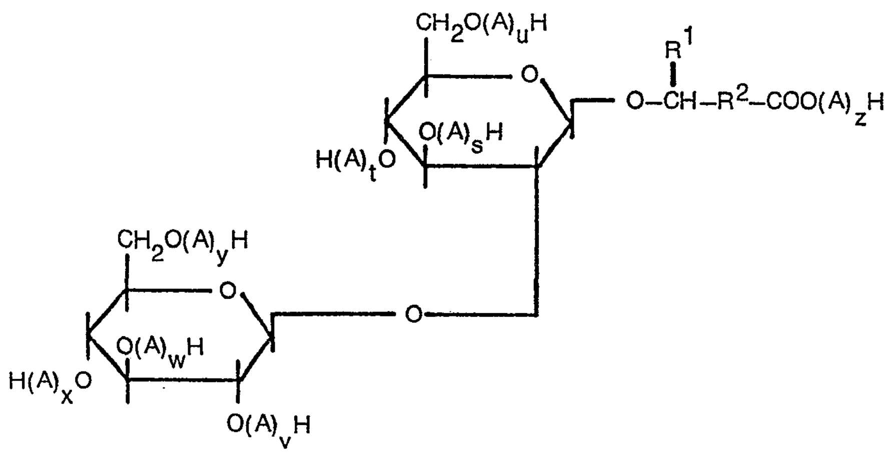

(II)

where $R^1$ is a methyl group or a hydrogen atom, $R^2$ is a straight-chain saturated or ethylenically unsaturated hydrocarbon group having 11 to 15 carbon atoms when $R^1$ is a methyl group, or a straight-chain saturated or ethylenically unsaturated hydrocarbon group having 12 to 16 carbon atoms when $R^1$ is a hydrogen atom, A is $—CH_2—CH_2—O—$ or

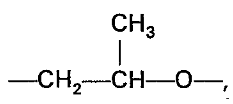

and s, t, u, v, w, x, y and z are integers, whose sum ranges from greater than 1 to 60, not all of the integers s to y being zero, z not being zero, and not all of the integers s to z having the value 1.

4. A hydroxyalkyl-etherified glycolipid ester according to Claim 3, characterised in that s, t, u, v, w, x, y and z are integers, whose sum ranges from 5 to 30.

5. A process for producing a hydroxyalkyl-etherified glycolipid ester as claimed in Claim 1, which comprises reacting a glycolipid ester represented by the formula (III):—

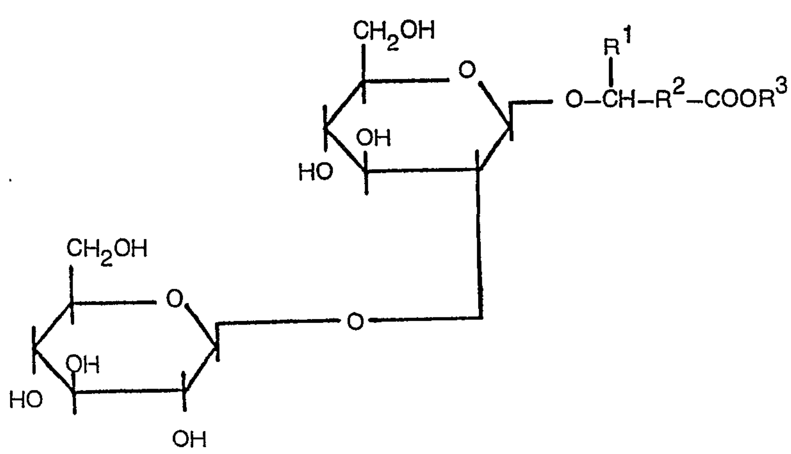

(III)

where $R^1$, $R^2$ and $R^3$ are the same as in formula (I), with ethylene oxide or 1,2-propylene oxide in the presence of at least one alkylene oxide addition catalyst.

6. A process for producing a hydroxyalkyl-etherified glycolipid ester as claimed in Claim 3, which comprises reacting a glycolipid represented by the formula (IV):

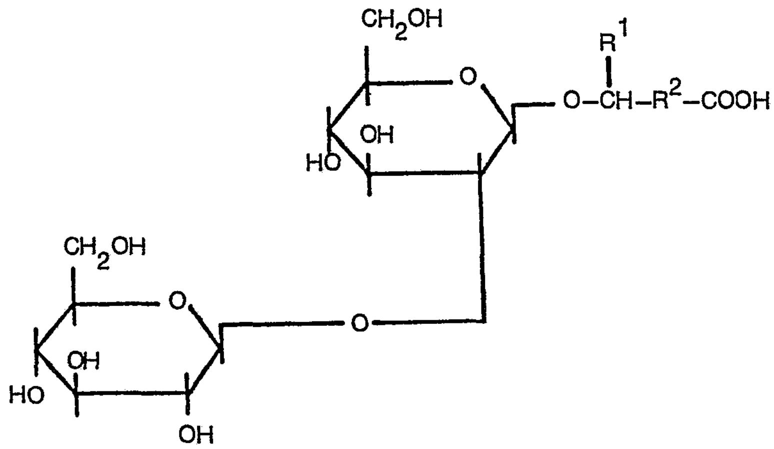

(IV)

where $R^1$ and $R^2$ are the same as in formula (II), with ethylene oxide or 1,2-propylene oxide in the presence of at least one alkylene oxide addition catalyst.

7. A process according to Claim 5 or Claim 6, characterised in that the alkylene oxide addition catalyst is metallic sodium, metallic potassium, sodium hydroxide, potassium hydroxide, a sodium alcoholate, or a potassium alcoholate.

8. A process according to any one of Claims 5 to 7, characterised in that reaction is conducted at a temperature of from 100 to 120°C.

9. A process according to any one of Claims 5 to 8, characterised in that the alkylene oxide addition catalyst is present in an amount of from 0.1 to 1% by weight of the glycolipid or glycolipid ester.

**Patentansprüche**

1. Ein durch Hydroxylalkylgruppen ätherifiertes Glycolipidester, das durch die Formel (I) dargestellt ist:

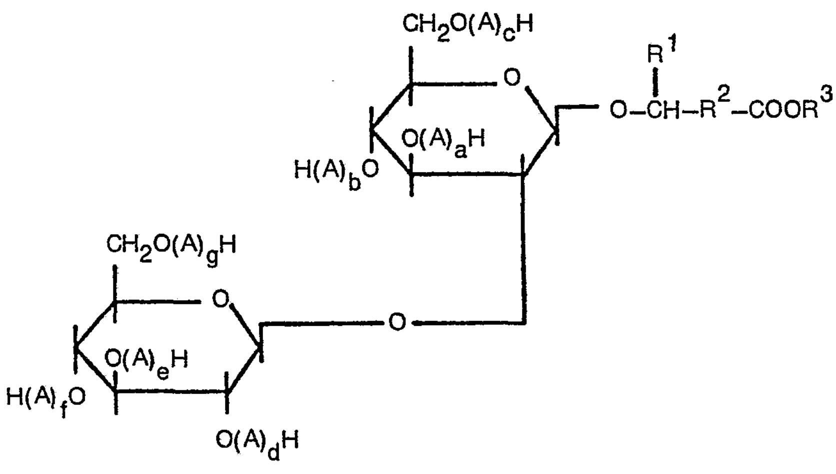

(I)

wobei $R^1$ eine Methylgruppe oder ein Wasserstoffatom ist und $R^2$ eine geradkettige, gesättigte oder äthylenungesättigte Kohlenwasserstoffgruppe mit 11 bis 15 Kohlenstoffatomen, wenn $R^1$ eine Methylgruppe ist oder eine geradkettige, gesättigte oder äthylenungesättigte Kohlenwasserstoffgruppe mit 12 bis 16 Kohlenstoffatomen, wenn $R^1$ ein Wasserstoffatom ist, und $R^3$ ist eine gesättigte oder ungesättigte Wasserstoffgruppe mit 1 bis 20 Kohlenstoffatomen, und A ist $—CH_2CH_2—O$ oder

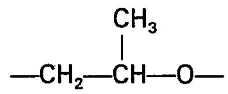

und a, b, c, d, e, f und g sind ganze Zahlen, deren Summe zwischen 1 und 60 liegt.

2. Ein durch Hydroxylalkylgruppen ätherifiertes Glycolipidester nach Anspruch 1, dadurch gekennzeichnet, daß a, b, c, d, e, f und g ganze Zahlen sind, deren Summe zwischen 5 und 30 liegt.

3. Ein durch Hydroxylalkylgruppen ätherifiertes Glycolipidester, das durch die Formel (II) dargestellt ist:

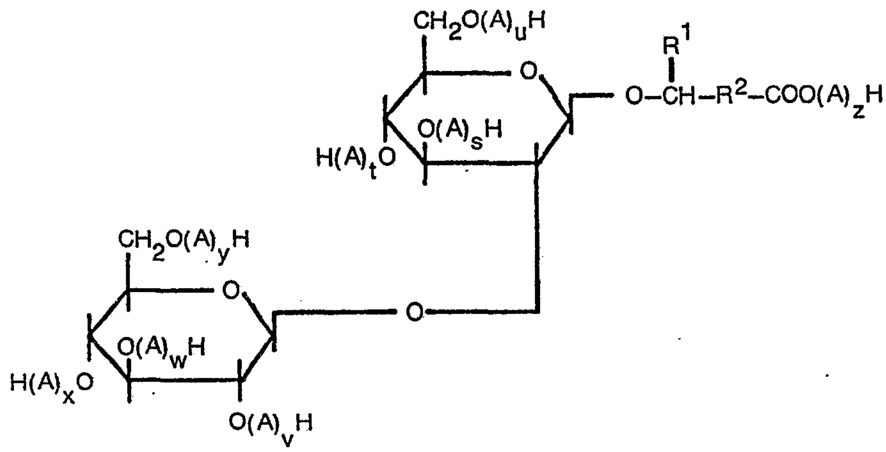

(II)

wobei $R^1$ eine Methylgruppe oder ein Wasserstoffatom ist, $R^2$ eine geradkettige, gesättigte oder äthylenungesättigte Kohlenwasserstoffgruppe mit 11 bis 15 Kohlenstoffatomen, wenn $R^1$ eine Methylgruppe ist, oder eine geradkettige, gesättigte oder äthylenungesättigte Kohlenwasserstoffgruppe mit 12 bis 16 Kohlenstoffatomen, wenn $R^1$ ein Wasserstoffatom ist, A ist $-CH_2-CH_2-O$ oder

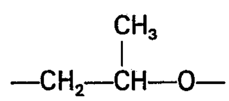

und s, t, u, v, w, x, y und z sind ganze Zahlen, deren Summe zwischen größer als 1 und 60 liegt, wobei nicht alle der ganzen Zahlen s bis y Null sind, z nicht Null ist und nicht alle der ganzen Zahlen s bis y den Wert 1 haben.

4. Ein durch Hydroxylalkylgruppen ätherifiertes Glycolipidester nach Anspruch 3, dadurch gekennzeichnet, daß s, t, u, v, w, x, y und z ganze Zahlen sind, deren Summe zwischen 5 und 30 liegt.

5. Ein Verfahren zur Herstellung eines durch Hydroxylalkylgruppen ätherifiertes Glycolipidesters nach Anspruch 1, welches die Reaktion eines Glycolipidesters einschließt, das durch die Formel (III) dargestellt ist:

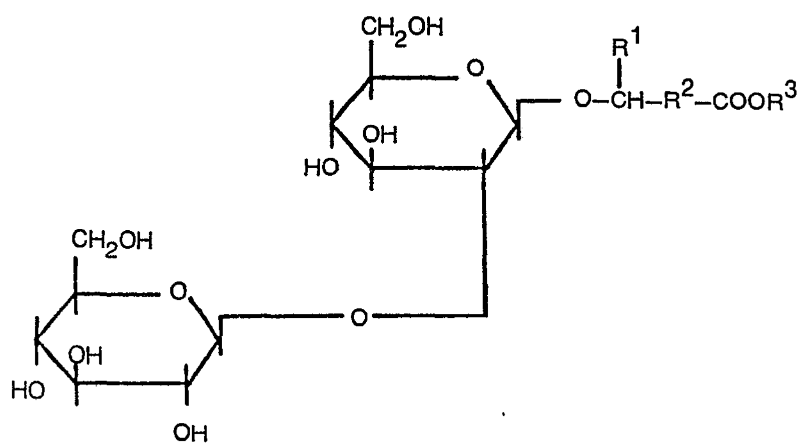

(III)

wobei $R^1$, $R^2$ und $R^3$ das gleiche bedeuten wie in Formel (I), mit Äthylenoxid oder 1,2-Propylenoxid in Gegenwart von mindestens einem Alkylenoxidzusatzkatalysator.

6. Ein Verfahren zur Herstellung eines durch Hydroxylalkylgruppen ätherifiertes Glycolipidesters nach Anspruch 3, welches eine Reaktion eines Glycolipids einschließt, das durch die Formel (IV) dargestellt ist:

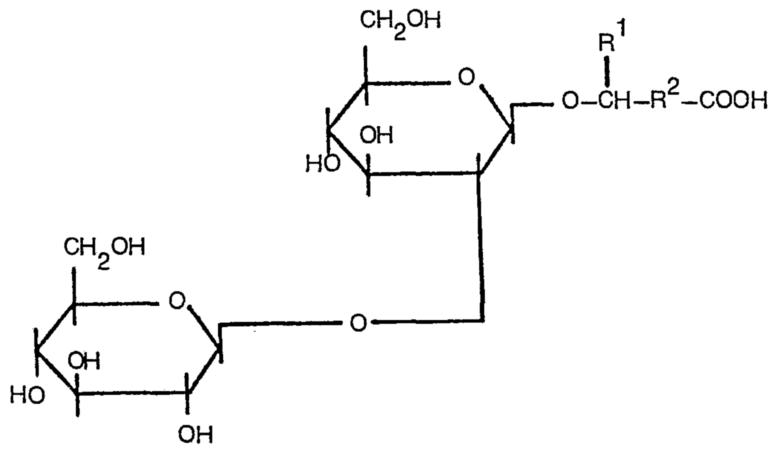

(IV)

wobei $R^1$ und $R^2$ das Gleiche bedeuten wie in Formel (II), mit Äthylenoxid oder 1,2-Propylenoxid in Gegenwart von mindestens einem Alkylenoxidzusatzkatalysator.

7. Ein Verfahren nach Anspruch 5 oder 6, dadurch gekennzeichnet, daß der Alkylenoxidzusatzkatalysator ein Natriummetall, ein Kaliummetall, ein Natriumhydroxid, ein Kaliumhydroxid, ein Natriumalkoholat oder ein Kaliumalkoholat ist.

8. Ein Verfahren nach einem der Ansprüche 5 bis 7, dadurch gekennzeichnet, daß die Reaktion bei einer Temperatur von 100 bis 120°C durchgeführt wird.

9. Ein Verfahren nach einem der Ansprüche 5 bis 8, dadurch gekennzeichnet, daß der Alkylenoxidzusatzkatalysator in einer Menge von 0,1 bis 1 Gew.-% Glycolipid oder Glycolipidester vorhanden ist.

**Revendications**

1. Ester glycolipidique hydroxyalkoyl-éthérifié représenté par la formule (I):

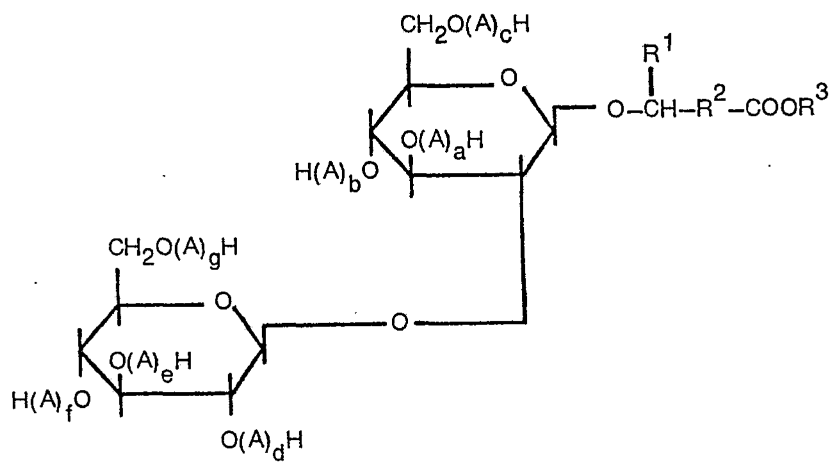

(I)

où $R^1$ est un groupe méthyle ou un atome d'hydrogène, $R^2$ est un groupe hydrocarbure à chaîne droite, saturé ou éthyléniquement insaturé possédant 11 à 15 atomes de carbone quand $R^1$ est un groupe méthyle, ou un groupe hydrocarbure à chaîne droite saturé ou électryléniquement insaturé possédant 12 à 16 atomes de carbone quand $R^1$ est un atome d'hydrogène, $R^3$ est un groupe hydrocarbure saturé ou insaturé possédant 1 à 20 atomes de carbone, A est $-CH_2-CH_2-O$ ou

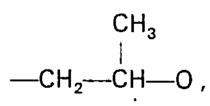

et a, b, c, d, e, f, et g sont des nombres entiers dont la somme se situe entre 1 et 60.

2. Ester glycolipidique hydroxyalcoyl-éthérifié suivant la revendication 1, caractérisé en ce que a, b, c, d, e, f, et g sont des nombres entiers dont la somme se situe entre 5 et 30.

3. Ester glycolipidique hydroxyalcoyl-éthérifié, représenté par la formule (II):

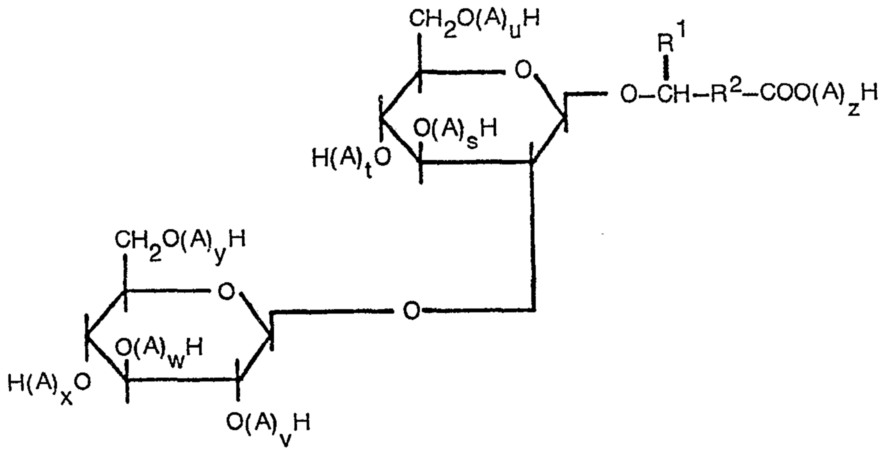

(II)

où $R^1$ est un groupe méthyle ou un atome d'hydrogène, $R^2$ est un groupe hydrocarbure à chaîne droite, saturé ou éthyléniquement insaturé possédant 11 à 15 atomes de carbone quand $R^1$ est un groupe méthyle, ou un groupe hydrocarbure à chaîne droite, saturé ou éthyléniquement insaturé possédant 12 à 16 atomes de carbone quand $R^1$ est un atome d'hydrogène, A est $-CH_2-CH_2-O-$ ou

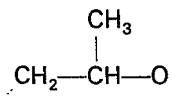

et s, t, u, v, w, x, y et z sont des nombres entiers dont la somme se situe de plus de 1 à 60, une partie seulement des nombres s à y étant zéro, z n'étant pas zéro, et une partie seulement des nombres entiers de s à z ayant la valeur 1.

4. Ester glycolipidique hydroxyalcoyl-éthérifié suivant la revendication 3, caractérisé en ce que s, t, u, v, w, x, y et z sont des nombres entiers dont la somme se situe entre 5 et 30.

5. Ester glycolipidique hydroxyalcoyle-éthérifié suivant la revendication 1, caractérisé en ce qu'on fait réagir un ester glycolipidique représentée par la formule (III):

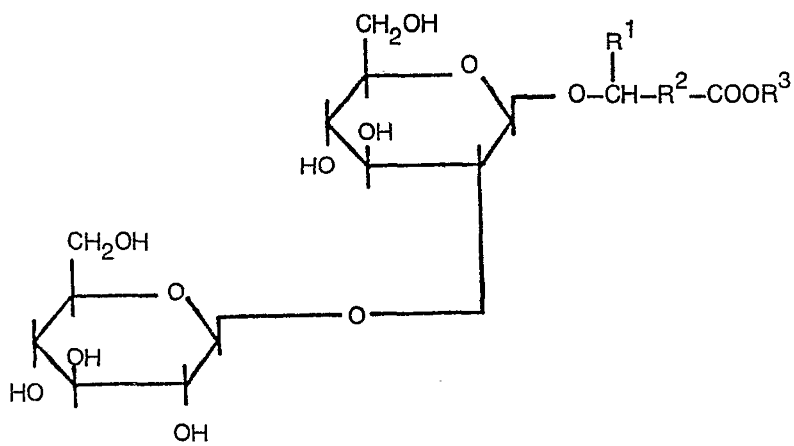

(III)

où $R^1$, $R^2$ et $R^3$ sont les mêmes que dans la formule (I), avec de l'oxyde d'éthylène ou de l'oxyde de 1,2-propylène, en présence d'au moins un catalyseur d'addition d'oxyde d'alkylène.

6. Procédé pour la production d'ester glycolipidique hydroxyalcoyl-éthérifié suivant la revendication 3, caractérisé en ce qu'on fait réagir un glycolipide représenté par la formule (IV):

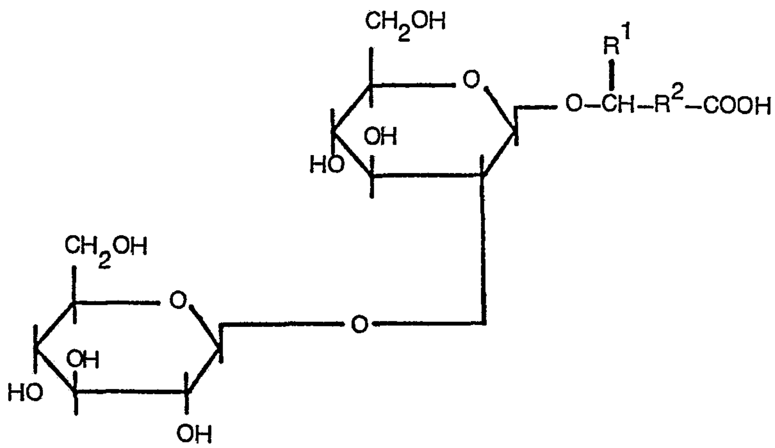

(IV)

où $R^1$ et $R^2$ sont les mêmes que dans la formule (II) avec de l'oxyde d'éthylène ou de l'oxyde de 1,2-propylène en présence d'au moins un catalyseur d'addition d'oxyde d'alkylène.

7. Procédé suivant l'une des revendications 5 et 6, caractérisé en ce que le catalyseur d'addition d'oxyde d'alkylène est constitué de sodium métallique, de potassium métallique, d'hydroxyde de sodium, d'hydroxyde de potassium, d'un alcoolate de sodium ou d'un alcoolate de potassium.

8. Procédé suivant l'une quelconque des revendications 5 à 7, caractérisé en ce que la réaction s'effectue à une température de 100 à 120°C.

9. Procédé suivant l'une quelconque des revendications 5 à 8, caractérisé en ce que le catalyseur d'addition d'oxyde d'alkylène est présent dans une proportion de 0,1 à 1% en poids calculé sur le poids glycolipide ou de l'ester glycolipidique.